# EUROPÄISCHE PATENTSCHRIFT

(11) **EP 1 318 970 B1**
(45) Veröffentlichungstag und Bekanntmachung des Hinweises auf die Patenterteilung: **29.11.2006**
(21) Anmeldenummer: 01972045.7
(22) Anmeldetag: 11.09.2001
(51) Int. Cl.: C07C 29/34, C07C 31/125

(54) **VERFAHREN ZUR HERSTELLUNG VON GUERBETALKOHOLEN**
METHOD FOR PRODUCING GUERBET ALCOHOLS
PROCEDE POUR PREPARER DES ALCOOLS DE GUERBET

(30) Priorität: 20.09.2000 DE 10046433
(43) Veröffentlichungstag der Anmeldung: 18.06.2003
(73) Patentinhaber: Cognis IP Management GmbH, 40589 Düsseldorf (DE)
(72) Erfinder: BOTH, Sabine, 40229 Düsseldorf (DE); FIEG, Georg, 40822 Mettmann (DE); REUTER, Erich, 40591 Düsseldorf (DE); BARTSCHICK, Frank, 41470 Neuss (DE); GUTSCHE, Bernhard, 40724 Hilden (DE)
(86) Internationale Anmeldenummer: PCT/EP2001/010477
(87) Internationale Veröffentlichungsnummer: WO 2002/024616

(56) Entgegenhaltungen:
- WO-A-91/04242

## Beschreibung

### Gebiet der Erfindung

Die Erfindung befindet sich auf dem Gebiet der kosmetischen Ölkörper und betrifft ein verbessertes Verfahren zur Herstellung von Guerbetalkoholen, welches ohne die Verwendung von Schwermetallkatalysatoren auskommt.

### Stand der Technik

Guerbetalkohole stellen in 2-Position verzweigte primäre Alkohole dar, welche durch Kondensation von linearen Fettalkoholen erhalten werden. Die Produkte finden überwiegend als Ölkomponenten zur Herstellung von kosmetischen Emulsionen Verwendung. Zu ihrer Herstellung geht man in der Regel von Fettalkoholen aus, die zunächst unter dem Einfluß starker Basen und Schwermetallverbindungen, wie beispielsweise Kupfer oder Zinkoxid selbst kondensieren. Man nimmt an, dass unter den Reaktionsbedingungen der Alkohol zunächst zum Aldehyd dehydriert wird, dieser eine Aldolkondensation mit sich selbst eingeht und das Kondensationsprodukt anschließend zum Alkohol hydriert wird. Eine Übersicht hierzu findet sich beispielsweise in **Angew.Chem. 64, 212 (1952).**

Ein Verfahren zur Herstellung von Guerbetalkoholen ist beispielsweise in WO 91/04242 beschrieben.

Von Nachteil ist jedoch, dass die Schwermetallkatalysatoren nach Beendigung der Reaktion wieder abgetrennt werden müssen, um gesetzliche Anforderungen zu erfüllen und sicherzustellen, dass sie nicht in der späteren Anwendung Irritationen hervorrufen. Die Abtrennung erfolgt in der Regel durch eine Wäsche mit anschließender Destillation, wobei diese mit nicht unerheblichen Produktverlusten verbunden ist.

Demzufolge hat die Aufgabe der vorliegenden Erfindung darin verstanden, ein Verfahren zur Herstellung von Guerbetalkoholen zur Verfügung zu stellen, welches verglichen mit dem Stand der Technik ökonomischer und mit verminderter Umweltbelastung arbeitet. Insbesondere sollte auf die Mitverwendung von Schwermetallkatalysatoren verzichtet werden können und eine einfache destillative Aufreinigung der Produkte ohne aufwendige Zentrifugenwäsche gewährleistet werden.

### Beschreibung der Erfindung

Gegenstand der Erfindung ist ein Verfahren zur Herstellung von Guerbetalkoholen der Formel **(I)**,

**CH**_{**3**}**(CH**_{**2**}**)**_{**n**}**CHR**^{**1**}**CH**_{**2**}**OH** **(I)**

in der R¹ für einen linearen Alkylrest mit n-1 Kohlenstoffatomen und n für Zahlen von 3 bis 9 Kohlenstoffatomen, bei dem man Fettalkohole der Formel **(II)**,

**R**^{**2**}**OH** **(II)**

in der R² für lineare Alkylreste mit 6 bis 12 Kohlenstoffatomen steht, in Gegenwart von Carbonylverbindungen und Alkalihydroxiden kondensiert,
wobei man die Carbonylverbindung bei einer Temperatur von 210 bis 240 °C zu der Mischung aus Fettalkoholen und Alkalihydroxid zudosiert, wobei man die Carbonylverbindung innerhalb von 10 bis 60 Minuten zugibt.

Überraschenderweise wurde gefunden, dass Carbonylverbindungen, speziell Fettaldehyde als Alternative zu Schwermetallverbindungen ebenfalls geeignete Katalysatoren für die Guerbetreaktion darstellen, insbesondere wenn sie bei hohen Temperaturen zugegeben werden. Demzufolge besteht ein besonderer Vorteil des Verfahrens darin, dass die Kondensation bei vergleichbaren Ausbeuten in Abwesenheit von Schwermetallen erfolgt, bei der Aufarbeitung keine Wäsche erforderlich ist und somit auch keine Produktverluste mehr auftreten.

### Fettalkohole

Für die Kondensation eignen sich Fettalkohole, vorzugsweise solche der Formel **(I)**, in der R² für einen Alkylrest mit 8 bis 10 Kohlenstoffatomen steht. Typische Beispiele sind Hexanol, Octanol, Decanol, Dodecanol sowie deren Gemische.

### Carbonylverbindungen

Als Carbonylverbindungen, die als Katalysatoren eingesetzt werden, kommen neben Ketonen vor allem Fettaldehyde in Frage, die vorzugsweise der Formel **(III)** folgen,

**R**^{**3**}**CHO** **(III)**

in der R³ für lineare Alkylreste mit 6 bis 12 und insbesondere 8 bis 12 Kohlenstoffatomen steht Typische Beispiele sind Hexanal, Octanal, Decanal, Dodecanal sowie deren Gemische. Als besonders vorteilhaft hat es sich erwiesen, Fettalkohole und Fettaldehyde mit gleichem Alkylrest einzusetzen. Üblicherweise werden die Carbonylverbindungen im allgemeinen und die Fettaldehyde im besonderen bezogen auf die Fettalkohole in Mengen von 0,2 bis 50, vorzugsweise 1 bis 25 und Insbesondere 3 bis 10 Mol-% eingesetzt.

### Kondensation

Die Kondensationsreaktion kann in an sich bekannter Weise durchgeführt werden, d.h. man legt die Fettalkohole zusammen mit den Basen vor und erhitzt sie. Die Carbonylverbindungen werden bei einer Temperatur von 210 bis 240 °C zu der Mischung aus Fettalkoholen und Alkalihydroxid zudosiert. Die Zugabe erfolgt innerhalb von 10 bis 60 min. Die Menge an Alkalihydroxiden kann 1 bis 10, vorzugsweise 3 bis 5 Mol-% bezogen auf die Fettalkohole betragen. Vorzugsweise wird mindestens 40 Gew.-%ige Natron- oder insbesondere Kalilauge verwendet Zur Verlagerung des Reaktionsgleichgewichtes auf die Produktseite empfiehlt sich in jedem Fall, das Kondensationswasser kontinuierlich abzudestillieren. Da hierbei leicht organisches Material mitgerissen wird, hat sich der Einsatz eines Dephlegmators bewährt, mit dessen Hilfe die organische Phase abgeschieden wird und in den Ansatz zurückgefahren werden kann. Die Aufarbeitung, die nun ohne Wäsche auskommt, kann anschließend durch einfache Destillation erfolgen. Durch diese Maßnahme fallen weniger Produktverluste und eine geringere Abwasserbelastung an.

### Beispiele

**Beispiel 1.** In einer Rührapparatur bestehend aus Kolben, Heizpilz, Dephlegmator, Liebigkühler, Stickstoffüberleitung und Doppelhubkolbenpumpe wurden 1000 g (6,3 Mol) Decanol (95 Gew.-%ig) vorgelegt, bei 20 °C mit 22,5 g (0,22 Mol) 45 Gew.-% Kaüumhydroxidlösung versetzt und bis auf 215 °C aufgeheizt. Das beim Ausheizen entstandene Wasser wurde kontinuierlich abdestilliert. Anschließend wurden über die Pumpe innerhalb von 60 min 29,6 g Decanal (entsprechend 3 Mol-% bezogen auf Decanol) zudosiert und die Temperatur dabei auf 240°C angehoben. Die destillierte organische Phase wurde nach Phasentrennung dem Reaktionsgemisch zugeführt. Nach 6 h war die Reaktion beendet. Eine GC-Analyse des Produktgemisches zeigte, dass 76 Gew.-% 2-Octyldodecanol entstanden waren; daneben befanden sich im Reaktionsgemisch 6 Gew.-% Trimere, 14 Gew.-% nicht umgesetzter Monomeralkohol und 4 Gew.-% gemischte Guerbetalkohole bestehend aus C₁₈- und C₂₂-Guerbetalkoholen. Die Aufreinigung des Produktes erfolgte destillativ, wobei das nicht umgesetzte Decanol als Vorlauf abgenommen und in die Reaktion zurückgeführt wurde; als Rückstand verblieben die höhermolekularen Bestandteile.

**Beispiel 2.** Analog Beispiel 1 wurden 1000 g (6,3 Mol) Decanol mit 22,5 (0,22 Mol) Kaliumhydroxidlösung und 29,6 g Decanal versetzt und auf 240 °C aufgeheizt. Das beim Aufheizen entstehende Wasser wurde kontinuierlich abdestilliert und die destillierte organische Phase nach Phasentrennung dem Reaktionsgemisch zugeführt. Nach 6 h war die Reaktion beendet. Eine GC-Analyse des Produktgemisches zeigte, dass 43 Gew.-% 2-Octyldodecanol entstanden waren; daneben befanden sich im Reaktionsgemisch 2 Gew.-% Trimere, 53 Gew.-% nicht umgesetzter Monomeralkohol und 2 Gew.-% gemischte Guerbetalkohole bestehend aus C₁₈- und C₂₂-Guerbetalkoholen.

## Patentansprüche

1. Verfahren zur Herstellung von Guerbetalkoholen der Formel **(I)**,
**CH**_{**3**}**(CH**_{**2**}**)**_{**p**}**CHR**^{**1**}**CH**_{**2**}**ON** **(I)**
in der R¹ für einen linearen Alkylrest mit n-1 Kohlenstoffatomen und n für Zahlen von 3 bis 9 Kohlenstoffatomen, bei dem man Fettalkohole der Formel **(II)**,
**R**^{**2**}**OH** **(II)**
In der R³ für lineare Alkylreste mit 6 bis 12 Kohlenstoffatomen steht in Gegenwart von Carbonylverbindungen und Alkalihydroxiden kondensiert, wobei man die Carbonylverbindungen bei einer Temperatur von 210 bis 240 °C zu der Mischung aus Fettalkoholen und Alkalihydroxid zudosiert, wobei man die Carbonylverbindung innerhalb von 10 bis 60 Minuten zugibt.

2. Verfahren nach Anspruch 1, **dadurch gekennzeichnet, dass** man Fettalkohole der Formel **(I)** einsetzt, in der R² für einen Alkylrest mit 8 bis 10 Kohlenstoffatomen steht.

3. Verfahren nach den Ansprüchen 1 und/oder 2, **dadurch gekennzeichnet, dass** man als Carbonylverbindungen Fettaldehyde einsetzt.

4. Verfahren nach mindestens einem der Ansprüche 1 bis 3, **dadurch gekennzeichnet, dass** man Fettaldehyde der Formel **(III)** einsetzt,
**R**^{**3**}**CHO** **(III)**
in der R³ für lineare Alkylreste mit 6 bis 12 Kohlenstoffatomen steht.

5. Verfahren nach mindestens einem der Anspruche 1 bis 4, **dadurch gekennzeichnet, dass** man die Carbonylverbindungen bezogen auf die Fettalkohole in Mengen von 0,2 bis 50 Mol-% einsetzt.

6. Verfahren nach mindestens einem der Ansprüche 1 bis 5 **dadurch gekennzeichnet, dass** man die Alkalihydroxide in Mengen von 1 bis 10 Mol-% bezogen auf die Fettalkohole einsetzt.

7. Verfahren nach mindeste einem der Ansprüche 1 bis 6, **dadurch gekennzeichnet, dass** man die Kondensationsprodukte anschließend durch Destillation reinigt

8. Verfahren nach mindestens einem der Anspruche 1 bis 7, **dadurch gekennzeichnet, dass** die Kondensation in Abwesenheit von Schwermetallkatalysatoren erfolgt.

## Claims

1. Process for the production of Guerbet alcohols corresponding to formula **(I)**:
**CH**_{**3**}**(CH**_{**2**}**)**_{**n**}**CHR**^{**1**}**CH**_{**2**}**OH** **(I)**
where R¹ is a linear alkyl group containing n-1 carbon atoms and n stands for numbers of 3 to 9 carbon atoms,
in which fatty alcohols corresponding to formula **(II):**
**R**^{**2**}**OH** **(II)**
where R² represents linear alkyl groups containing 6 to 12 carbon atoms, are condensed in the presence of carbonyl compounds and alkali metal hydroxides, the carbonyl compounds being added to the mixture of fatty alcohols and alkali metal hydroxide over a period of 10 to 60 minutes at a temperature of 210 to 240°C.

2. Process as claimed in claim 1, **characterized in that** fatty alcohols corresponding to formula (I), where R² is an alkyl group containing 8 to 10 carbon atoms, are used.

3. Process as claimed in claims 1 and/or 2, **characterized in that** fatty aldehydes are used as the carbonyl compounds.

4. Process as claimed in at least one of claims 1 to 3, **characterized in that** fatty aldehydes corresponding to formula **(III)**:
**R**^{**3**}**CHO** **(III)**
where R³ represents linear alkyl groups containing 6 to 12 carbon atoms, are used.

5. Process as claimed in at least one of claims 1 to 4, **characterized in that** the carbonyl compounds are used in quantities of 0.2 to 50 mol-%, based on the fatty alcohols.

6. Process as claimed in at least one of claims 1 to 5, **characterized in that** the alkali metal hydroxides are used in quantities of 1 to 10 mol-%, based on the fatty alcohols.

7. Process as claimed in at least one of claims 1 to 6, **characterized in that** the condensation products are purified by distillation.

8. Process as claimed in at least one of claims 1 to 7, **characterized in that** the condensation is carried out in the absence of heavy metal catalysts.

## Revendications

1. Procédé pour préparer des alcools de Guerbet de formule (I),
CH₃(CH₂)ₙCHR¹CH₂OH (I)
dans laquelle R¹ représente un radical alkyle linéaire ayant n-1 atomes de carbone et n représente des nombres de 3 à 9 atomes de carbone, selon lequel
on condense des alcools gras de formule (II),
R²OH (II)
dans laquelle R² représente des radicaux alkyle linéaires ayant 6 à 12 atomes de carbone, en présence de composés carbonyle et d'hydroxydes alcalins, les composés carbonyle étant ajoutés en les dosant au mélange d'alcools gras et d'hydroxyde alcalin, à une température de 210 à 240 °C et le composé carbonyle étant ajouté en l'espace de 10 à 60 minutes.

2. Procédé selon la revendication 1,
**caractérisé en ce que**
l'on emploie des alcools gras de formule (I) dans laquelle R² représente un radical alkyle ayant 8 à 10 atomes de carbone.

3. Procédé selon les revendications 1 et/ou 2,
**caractérisé en ce qu'**
on emploie des aldéhydes gras en tant que composés carbonyle.

4. Procédé selon au moins l'une des revendications 1 à 3,
**caractérisé en ce qu'**
on emploie des aldéhydes gras de formule (III),
R³CHO (III)
dans laquelle R³ représente des radicaux alkyle linéaires ayant 6 à 12 atomes de carbone.

5. Procédé selon au moins l'une des revendications 1 à 4,
**caractérisé en ce qu'**
on emploie les composés carbonyle, dans des quantités allant de 0,2 à 50 % en moles par rapport aux alcools gras.

6. Procédé selon au moins l'une des revendications 1 à 5,
**caractérisé en ce qu'**
on emploie les hydroxydes alcalins dans des quantités allant de 1 à 10 % en moles par rapport aux alcools gras.

7. Procédé selon au moins l'une des revendications 1 à 6,
**caractérisé en ce qu'**
on purifie ensuite les produits de condensation par distillation.

8. Procédé selon au moins l'une des revendications 1 à 7,
**caractérisé en ce que**
la condensation s'effectue en l'absence de catalyseurs à base de métaux lourds.
